(19) **Europäisches Patentamt European Patent Office Office européen des brevets**

(11) **EP 4 244 331 B1**

(12) **EUROPEAN PATENT SPECIFICATION**

(45) Date of publication and mention of the grant of the patent:
**17.07.2024 Bulletin 2024/29**

(21) Application number: **21856899.6**

(22) Date of filing: **17.12.2021**

(51) International Patent Classification (IPC):
**C12M 1/00** *(2006.01)*   **C12M 1/21** *(2006.01)*
**C12M 1/34** *(2006.01)*

(52) Cooperative Patent Classification (CPC):
**C12M 23/34; C12M 23/58; C12M 29/06; C12M 41/02; C12M 41/26; C12M 41/34**

(86) International application number:
**PCT/CZ2021/050153**

(87) International publication number:
**WO 2022/127952 (23.06.2022 Gazette 2022/25)**

(54) **A MULTI-CHAMBER NITRIFICATION REACTOR FOR FUGATE PROCESSING AND METHOD OF ITS START-UP INTO FULL SCALE OPERATION**

MEHRKAMMER-NITRIFIKATIONSREAKTOR ZUR FUGATE-VERARBEITUNG UND VERFAHREN ZU DESSEN INBETRIEBNAHME IN VOLLMASSSTABBETRIEB

RÉACTEUR DE NITRIFICATION MULTI-CHAMBRE POUR LE TRAITEMENT D'EAUX DE TRAITEMENT DE TYPE « FUGATE » ET SON PROCÉDÉ DE DÉMARRAGE EN FONCTIONNEMENT À GRANDE ÉCHELLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2020 CZ 202038384 U**
**18.12.2020 CZ 20200694**

(43) Date of publication of application:
**20.09.2023 Bulletin 2023/38**

(73) Proprietor: **Ceská zemedelská univerzita v Praze 16521 Praha 6 (CZ)**

(72) Inventors:
• **SVEHLA, Pavel**
**14000 Praha 4 - Krc (CZ)**
• **MICHAL, Pavel**
**16900 Praha 6 - Brevnov (CZ)**
• **TLUSTOS, Pavel**
**14000 Praha 4 - Krc (CZ)**

(74) Representative: **Harber IP s.r.o.**
**Dukelskych hrdinu 567/52**
**17000 Praha 7 (CZ)**

(56) References cited:
**CN-A- 106 520 610    DE-A1- 102007 005 069**

• **SVEHLA PAVEL ET AL: "Nitrification in a completely stirred tank reactor treating the liquid phase of digestate: The way towards rational use of nitrogen", WASTE MANAGEMENT, ELSEVIER, NEW YORK, NY, US, vol. 64, 3 April 2017 (2017-04-03), pages 96 - 106, XP085047974, ISSN: 0956-053X, DOI: 10.1016/J.WASMAN.2017.03.041**

## Description

Field of Art

The present invention relates to a unique multi-chamber design of a nitrification reactor capable of processing, within a given agricultural biogas plant, the entire production of fugate, or a required part thereof. The fugate is produced in buildings of biogas plants equipped with a digestate separator. It represents the liquid fraction of the digestate, while during the separation it is formed together with the so-called separate, which is the solid fraction of the digestate. It has been shown that the treatment of fugate by the biological nitrification process can lead to the elimination of a number of problems, in particular to the prevention of nitrogen losses during the handling of fugate.

Background Art

The fugate is currently usually applied directly to agricultural land as a source of nutrients for plant nutrition. It contains mainly nitrogen and potassium compounds, the content of other nutrients, including phosphorus, can also be significant. However, the big problem is that nitrogen, as essentially the most valuable nutrient contained in fugate, occurs in a relatively unstable form there. Most of the nitrogen in the fugate is present in the form of total ammonia nitrogen (TAN, the sum of $N-NH_4^+$ and $N-NH_3$). Depending on the environmental conditions, TAN occurs in two dissociation forms, as $N-NH_4^+$ and $N-NH_3$. The representation of these two forms depends mainly on the pH value and the temperature. There is a relatively high proportion of $N-NH_3$ in the fugate environment, which is volatile. This results in a significant risk of $NH_3$ escape into the atmosphere, and thus the loss of valuable nutrient and environmental pollution during handling of the fugate, especially during its storage and directly during its application to agricultural land. In recent years, much attention has been paid to research in the field of fugate processing methods, which can guarantee the elimination of the problems outlined above. It has been shown that a promising option is to convert the unstable form of nitrogen (TAN) to a substantially more stable form ($N-NO_3^-$) by initiating the biological nitrification process in the fugate environment. Assuming a combination of nitrification of the fugate with subsequent thermal thickening of the nitrified fugate, there may also be savings in the costs of storing the fugate and transporting it from the biogas plant to agricultural land (Svehla et al., 2020, Journal of Environmental Management. 276. 111250).

CN106520610A relates to production technology of high-density composite nitrobacterium inoculant, and high-density fermentation batch culture fermentation tank system of nitrobacteria. The disclosed high-density batch culture production technology of a nitrobacterium inoculant for degrading ammonia nitrogen in water, comprises the steps of magnetic nano-powder preparation, nitrobacterium seed liquid culture, magnetically carried nitrobacterium immobilization, batch fermentation culture, flocculation mixed precipitate magnetic separation, freeze drying and preparation compounding. The production technology improves the effective cell density of a nitrobacterium preparation, allows a flocculating agent and a stimulant to be compounded while preparing the nitrobacteria inoculant, and the nitrobacterium inoculant can be used in water restoration, can improve the transparence of water, and guarantees the sufficient nitrobacterium cell density required by water restoration and adaptability to new environment.

The high-density fermentation batch culture fermentation tank system of nitrobacteria, used for the technology, contains one ventilated mixing tank with a feeding pump, discharging pump, flocculant dosing tube, pH adjustment means, a dosing tube for defoaming agent, and a stirrer. Dissolved oxygen and pH are also measured during the process.

Disclosure of the Invention

Due to the extreme conditions prevailing in the environment of the fugate, which are very far from the optimal conditions in terms of the activity of nitrifying organisms, it is necessary to choose a suitable strategy for initiating the nitrification process in this material (Svehla et al., 2017, Waste Management. 64. 96-106). This may consist, for example, in the use of a device for the cultivation of nitrifying biomass, which processes fugate, from which the necessary resistant culture of microorganisms needed to inoculate a full scale reactor capable of processing all or required part of the fugate production within a given biogas plant is obtained.

However, due to the limited amount of biomass produced by the device for cultivation of nitrifying biomass, it is necessary to determine a suitable procedure to launch operation of the reactor capable of processing the entire production of fugate in the conditions of a given biogas plant and define its parameters crucial for trouble-free start-up of the process in full scale conditions.

During the start-up of fugate processing reactor, the fugate being characterized by an extremely high concentration of TAN, there is a risk of inhibition of nitrifying organisms due to toxic nitrogen compounds. Although TAN is a substrate for the organisms of the first phase of nitrification - ammonia oxidizing bacteria (AOB), it has an inhibitory effect on them at higher concentrations. The process of start-up of the system, which is usually carried out in a single-chamber nitrification reactor (addition of a smaller amount of inoculum to a significantly larger volume of treated water) would therefore lead to inhibition or even killing of AOB. This is especially true at the higher pH values typical for fugate due to the increase in the proportion of toxic $NH_3$ in the TAN. Even more sensitive than AOB are the organisms of the second phase of nitrification - nitrite oxidizing bacteria (NOB). Their inhibition can then cause the accumulation of nitrite in the system, which can lead to even stronger inhibition of AOB and especially NOB. Thanks tothe presence of at least three separate chambers in the nitrification reactor, startup procedures can be followed in which no TAN accumulation occurs, as it is immediately converted to nontoxic $N-NO_3^-$ by AOB and NOB. For the same reason, nitrite accumulation cannot occur either.

*Definitions*

TAN = Total ammonia nitrogen;
fugate = liquid phase of the digestate produced within the biogas plants operation;
target nitrogen loading rate (Bv) = nitrogen loading rate of the reactor in kg of TAN entering 1 $m^3$ of the reactor in 1 day during the treatment of the entire production of fugate, or a required part thereof;
target pH = pH value optimal for long-term operation of nitrification reactor constructed for fugate treatment in concrete biogas plant;
inoculum = a mixed microbial culture used with the aim to supply nitrification organisms; nitrifying biomass = a mixed microbial culture cultivated in the form of suspension containing nitrification organisms able to be highly active in the environment of fugate.

The object of the present invention is a nitrification reactor for fugate processing, which comprises:

- a tank which is divided into three separate chambers, wherein the chambers have mutually different volumes, wherein the volume of the first chamber is smaller than the volume of the second chamber, which is smaller than the volume of the third chamber, said tank comprises an air distribution device connected to a source of compressed air for aerating all the chambers of the tank;
- a measuring and control unit located outside the tank;
- an inlet for raw fugate into each of the tank chambers;
- an outlet for the nitrified fugate from each of the tank chambers;
- a probe for measuring pH, located inside each chamber and connected to the measuring and control unit;
- a probe for measuring the $O_2$ concentration, located inside each chamber and connected to the measuring and control unit;
- an inlet for a defoamer in each of the chambers, connected to a pump connected to a container for defoamer;
- a neutralization station, located outside the tank, comprising a tank for the pH adjusting agent and a pump, said neutralization station being connected to each chamber by an inlet for the pH adjusting agent;

wherein the pump, and the air distribution device, eventually the neutralization station, are connected to the measuring and control unit by feedback; and wherein the volume of the first chamber is in the range of from 5 to 10% of the total reactor tank volume, the volume of the second chamber is in the range of from 20 to 30% of the total tank volume, and the volume of the third chamber is in the range of from 60 to 75% of the total volume of the nitrification reactor tank; and wherein the device further comprises a pump configured to pump the liquid content from the first chamber to the second chamber and from the second chamber to the third chamber.

The pH adjusting agent for the neutralization station is preferably an aqueous solution of NaOH or $Ca(OH)_2$, which is suitable for adjusting the pH of the processed fugate, however, other alternative basic agents can be used for pH adjustment, especially aqueous solutions of alkali metal or alkaline earth metal hydroxides.

The tank of the nitrification reactor is preferably made of concrete or plastic and preferably has a volume in the range of from 30 to 500 $m^3$. The nitrification reactor tank is divided into three separate chambers of unequal volume. The chambers are preferably located horizontally next to each other (preferably the nitrification reactor tank is divided by concrete, plastic or other partitions into three separate chambers situated next to each other) and each chamber is separate (the nitrification process takes place in each chamber independently of the other chambers). Each chamber has a separate inlet for raw fugate and an outlet for nitrified fugate, its own air distribution and probes for measuring pH and $O_2$. During regular operation, the fugate does not pass from one chamber to another; however, in the event of an 'accident' or problem, nitrifying biomass from one chamber can be used to 'revive' nitrifying bacteria in the chamber in which the problem occurred. Each chamber works in conditions approaching the so-called completely stirred tank reactor (CSTR), i.e. in the whole volume of the chamber there is a mixture with practically identical properties. The stirring of the content of the chamber is ensured by air which bubbles in the entire volume of the chamber. Thus, the inlet for raw fugate and the outlet for nitrified fugate can be located above the air distribution device at any height of the chamber.

In a preferred embodiment, the tank or each of its chambers comprises a separate inlet for nitrifying biomass. This makes it possible to continuously add to each chamber nitrifying biomass produced, for example, in the nitrifying biomass cultivation device described below.

The optimum volume of the nitrification reactor for a particular biogas plant is preferably determined based on the concentration of TAN in raw fugate to be nitrified and the target volume nitrogen loading rate of the reactor. The target nitrogen loading rate is determined in advance within the laboratory simulation of the whole nitrification process. In a small laboratory reactor (volume approx. 1 to 5 L) the nitrification process is initiated and operated analogously to the described reactor according to the present invention (nitrogen loading rate and pH at the start of nitrification depends on the inoculum of nitrifying microorganisms used and the source of the fugate - its chemical composition). Preferably, activated sludge from a municipal wastewater treatment plant is used for inoculation. Gradual regulation of conditions, especially fugate flow rate, determines what loading rate the system is able to process under the given conditions (i.e. nitrogen loading rate at which nitrifying microorganisms are effectively cultivated where no risk of overloading of the system and from it resulting deterioration of the function of the reactor could be expected). Too high fugate flow rate and/or too low pH in the reactor would lead to undesired disruption of the nitrifying microorganism cultivation process. The rate of dosing of the fugate into the nitrification reactor is determined by the required nitrogen loading rate of the device. It depends on the target nitrogen loading rate and the concentration of TAN in the processed fugate. The nitrogen loading rate is calculated multiplying the nitrogen concentration in the fugate and the fugate flow rate, divided by the tank volume.

The target nitrogen loading rate is preferably in the range of from 0.3 to 0.8 kg/(m$^3$·d); the target operating pH value is then in the range of from 4.5 to 6.

The nitrification reactor tank is divided into 3 chambers (S 1, S2 and S3), the volume of the first chamber S 1 is smaller than the volume of the second chamber S2, which is smaller than the volume of the third chamber S3,

the volume of the first chamber S 1 representing 5 to 10 per cent of the total reactor tank volume, the volume of the second chamber S2 representing 20 to 30 per cent of the total reactor tank volume and the volume of the third chamber S3 representing 60 to 75 per cent of the total volume of the nitrification reactor tank. In the most preferred embodiment, S1, S2 and S3 occupy 8.5, 25.5 and 66 per cent of the total volume of the nitrification reactor tank.

In one embodiment, the source of compressed air is selected from the group comprising a blower and a compressor. The air distribution device is preferably located at the bottom of each chamber of the reactor tank, more preferably over the entire area of the tank chamber bottom, and ensures homogenization of the suspension throughout its volume and prevents its sedimentation. In one embodiment, the air distribution device can be, for example, a perforated material, a perforated tube or nozzles (e.g. suitably perforated hose, preferably made of flexible material, the openings of which close themselves at rest, perforated or porous surface, etc.).

The transport of the fugate to the nitrification reactor tank and the outflow of the nitrified fugate from the nitrification reactor tank are preferably provided by pumps used in wastewater treatment. These are mainly sludge, submersible, self-priming, centrifugal, horizontal/vertical or spiral body pumps.

It is necessary to ensure continuous measurement of pH and oxygen concentration within the individual chambers of the nitrification reactor. At the same time, it is necessary to install a system capable of maintaining the required pH value (dosing of pH adjusting agent from the neutralization station) and oxygen concentration (control of compressed air supply intensity) in the reactor chambers by a pH probe and an $O_2$ probe, which are located inside each tank chamber and connected to the measuring and control unit. It is possible to use common measuring and control systems available on the market. The pH value is adjusted by adding a pH adjusting agent by a pump from the tank. The pH adjusting agent may be selected from the group comprising aqueous alkali metal hydroxide solutions, $CaCO_3$, $Ca(OH)_2$.

In one preferred embodiment, a source of compressed air is also connected to the measuring and control unit by feedback, which ensures a smooth and automatic control of the aeration intensity depending on the measured value of the $O_2$ concentration in the fugate.

In order to prevent increased foaming during aeration of the fugate in the reactor tank, each of the tank chambers comprises an inlet for defoamer connected to the container for defoamer. Through this inlet, the defoamer is dosed from the container for defoamer to the individual chambers of the reactor tank. More preferably, the container for defoamer is connected to a dispenser which is connected to the measuring and control unit by feedback. This makes it possible to automate the defoamer dosing into the tank.

Another object of the present invention is a method of start-up (putting into operation) of a multi-chamber nitrification reactor according to the present invention. First, laboratory tests determine the target nitrogen loading rate of nitrification reactor of the given biogas plant and the operating pH value for the nitrification reactor. These laboratory tests are performed before the actual installation of the nitrification reactor according to the present invention in the area of the given biogas plant.

The process is simulated in the laboratory in a small reactor with a volume of 1 to 5 L. The whole nitrification process is simulated, the conditions are varied (the fugate flow rate and thus nitrogen loading rate, and other parameters - pH, $O_2$ concentration) and the efficiency of the nitrification process is checked (the proportion of nitrified nitrogen in the effluent fugate is determined). Depending on the efficiency of the process, the conditions (target nitrogen loading rate and operating pH value) ideal for the fugate from the given biogas plant are selected (i.e. those conditions under which the proportion of nitrified nitrogen in the effluent fugate is maximum). As the flow rate of the fugate increases, after exceeding a certain concentration of TAN and a certain pH value, the cultivation of nitrifying biomass decreases or stops, and thus the production of nitrified nitrogen decreases. Due to the variable properties of the fugate, the optimum conditions during processing material from different biogas plants can vary significantly, so it is necessary to determine the target loading rate of the nitrification reactor and the pH for each biogas plant. The target loading rate of the nitrification reactor depends not only on the concentration of TAN in raw fugate, but also on the current fugate flow rate and the nitrification reactor volume. It is given in kg of TAN entering the 1 m³ tank in one day. Thus, the laboratory simulation defines the combination of N-ammon concentration and fugate flow rate that the reactor of a given volume 'manages' .

The target nitrogen loading rate of the system and the average TAN concentration in the fugate will then make it possible to determine the reactor volume for fugate processing so that the required hydraulic retention time for the fugate in the reactor is achieved. The hydraulic retention time ($\Theta$) in days is calculated according to the formula as the ratio of the concentration (c) of TAN (kg/m³) and the target nitrogen loading rate Bv (kg/(m³·d)) according to the formula $\Theta = c/Bv$.

The reactor volume is calculated according to equation (1) from the 'target nitrogen loading rate', the daily production of fugate (i.e. the daily amount of raw fugate processed) and the concentration of TAN in raw fugate.

$$V = c \cdot Q/Bv \quad (1),$$

where V is the volume of the nitrification reactor (m³); Bv is the target nitrogen loading rate (kg/(m³·d)), determined by the laboratory simulation of the nitrification process described above; c is the concentration of TAN in raw fugate (kg/m³); Q is the target flow rate of fugate through the reactor (or daily production of raw fugate within a given biogas plant, respectively) (m³/d).

Subsequently, the nitrification reactor according to the present invention is constructed, the tank of which is divided into at least three separate chambers of unequal size. Preferably the volume of the first chamber (S 1) is from 5 to 10 per cent of the total tank volume; the volume of the second chamber (S2) is from 20 to 30 per cent of the total tank volume; and the volume of the third chamber (S3) is from 60 to 75 per cent of the total volume of the nitrification reactor tank. Most preferably the volume of the first chamber (S1) is 8.5 per cent of the total tank volume; the volume of the second chamber (S2) is 25.5 per cent of the total tank volume; and the volume of the third chamber (S3) is 66 per cent of the total volume of the nitrification reactor tank.

Subsequently, a nitrifying culture of microorganisms capable of processing the fugate is placed into the first chamber S 1 of the nitrification reactor tank, which has a volume corresponding to the value determined above. Preferably, the nitrifying culture of microorganisms (capable of processing the fugate) is placed into the first chamber S 1 in an amount of 10 to 50 per cent of the volume of the first chamber S 1 of the tank.

In a preferred embodiment, the nitrifying biomass cultivated in the nitrifying biomass cultivating device described below is used, which is placed into the first chamber S 1 in an amount of 10 to 50 per cent of the volume of the first chamber S 1 of the tank. Furthermore, the raw fugate produced by the given biogas plant is gradually fed into the first chamber S1 at a daily inflow rate of from 0.25 to 5 per cent of the total daily fugate production of the given biogas plant. Preferably, the same amount of nitrifying culture of microorganisms, preferably produced by the nitrifying biomass cultivation device described below, is dosed into S1 at the same time.

The amount of raw fugate dosed into S 1, or the intensity of its inflow, gradually increases so as to maintain the constant target nitrogen loading rate. The current inflow intensity is determined by relation (2):

$$Q = Bv \cdot V/c \quad (2),$$

wherein V is the current volume of the mixture in the given chamber (m³); Bv is the target nitrogen loading rate (kg/(m³·d)); c is the concentration of TAN in the fugate (kg/m³) and Q is the current intensity of fugate flow rate through the reactor (or daily production of fugate within the given biogas plant) (m³/d). Preferably, the effluent from the nitrifying biomass cultivation device is also dosed into S 1 in a constant amount. During the process described above, the nitrifying culture of microorganisms, supplied to the mixture with the fugate, preferably from the nitrifying biomass cultivation device described below, gradually propagates (the fugate represents a substrate for the nitrifying microorganisms). The addition of further raw fugate creates a new nitrifying biomass that needs a new fresh substrate - in this case further raw fugate. Therefore, it is necessary to gradually increase the fugate dosage.

The pH value is maintained at all times at the operating pH value specified above. Typically, the pH value is in the range of from 4.5 to 6.

In a preferred embodiment, the gradual increase in the raw fugate dosage is carried out in five-day periods, so that five days after the inoculation, the volume of fugate in the first chamber S1 corresponds to 11 to 75 per cent of the total volume of S1. The raw fugate dosage is constant for five days and is given by equation (2), so the volume of the mixture inside the chamber S1 increases according to the dosing rate of the raw fugate. After five days of constant rate of dosing raw fugate into chamber S1, the dosing rate increases (calculated from equation (2) based on the current volume of the mixture in the chamber at the end of the previous five-day period), and for the following five days the raw fugate is dosed to S1 at this higher flow rate. The increase of the dosing rate continues in five-day cycles so that the target nitrogen loading rate is not exceeded, until the chamber S1 is completely filled with the mixture of fugate and nitrifying biomass. The purpose of this gradual increase in dosing is to speed up the nitrification reactor start-up process. If the raw fugate were dosed at the original rate throughout the reactor start-up, this start-up would take a longer time.

At the moment of filling up the entire first chamber S1, all the liquid contained in S1 is pumped into the second chamber S2, and the gradual filling of the second chamber S2 begins. The dose of raw fugate continues to increase according to relation (2) above. In the next five-day periods, the daily flow rate of raw fugate gradually increases. As soon as the entire second chamber S2 is filled up, all the liquid contained in S2 is pumped into the third chamber S3 and the gradual filling of the chamber S3 begins, analogously to the chambers S1 and S2. The dose of raw fugate continues to increase according to relation (2) above.

When the third chamber S3 is filled up, a part of the mixture from S3 is transferred to S1 and S2 so that the volume of liquid in all chambers represents the same part of their total capacity (under given conditions from 60 to 75 per cent of the capacity of each chamber). From this point on, the raw fugate is fed to the individual chambers in an amount corresponding to their share in the total volume of the operating reactor. Thus, if the chamber S1 occupies from 5 to 10 per cent of the volume of the entire nitrification reactor tank, then from 5 to 10 per cent of the volume of the total raw fugate to be processed will be fed into it. If the chamber S2 occupies from 20 to 30 per cent of the volume of the entire nitrification reactor tank, then from 20 to 30 per cent of the volume of the total raw fugate to be processed will be fed into it, and if S3 occupies from 60 to 75 per cent of the volume of the entire nitrification reactor tank, then about from 60 to 75 per cent of the total amount of raw fugate fed to the system will be fed to it. At the same time, according to formula (2), the dosing of raw fugate will continue to increase in five-day periods, until it is gradually increased to a value corresponding to the total daily fugate production of the given biogas plant, or its required part. This will achieve the processing of the required amount of fugate produced by the given biogas plant and the nitrification reactor start-up will be considered as completed (the reactor will be ready for full scale operation).

Once all the chambers of the nitrification reactor tank have been filled up, the nitrified fugate leaving the reactor can be used for application to the soil as fertilizer. In a preferred embodiment, the cultivated nitrifying biomass is added to enrich the system with nitrifying bacteria throughout the process of start-up of the full scale reactor After all the chambers of the nitrification reactor have been filled up, this addition of nitrifying bacteria can be stopped, because the amount of nitrifying organisms coming with it to the full scale reactor is already negligible in terms of the total volume of the fully operating reactor.

In a preferred embodiment, a nitrifying biomass cultivation device can be used to inoculate the nitrification reactor for fugate processing. Within this device, the necessary nitrifying culture of microorganisms capable of processing the fugate under the conditions defined in the previously performed laboratory experiments is obtained. Said nitrifying biomass cultivation device comprises a plastic tank with a volume in the range of from 0.5 to 2 m³, which comprises at least one aeration element connected to a source of compressed air; an inlet for biomass located in the lower third of the tank height; an outlet for nitrified biomass, preferably located in the upper third of the tank height; a pH measuring probe and an $O_2$ concentration measuring probe located inside the tank and connected to the measuring and control unit; and an inlet for pH adjusting agent connected to a pump connected to the pH adjusting agent tank, wherein the pump and the aeration element are connected to the measuring and control unit by feedback. The device may further comprise an inlet for defoamer connected to a container for defoamer.

The required nitrifying culture of microorganisms capable of processing the fugate is obtained in the described device as follows: the tank of the device is first filled with inoculum, for example activated sludge collected in the biological stage of a municipal wastewater treatment plant can be used. The dosing of the fugate into the tank of the device is then started through the substrate inlet, preferably by means of a peristaltic pump. The mixture of the inoculum and fugate is aerated in the tank by means of at least one aeration element connected to a source of compressed air. The dosing and aeration rate is regulated by a measuring and control unit depending on the pH and $O_2$ concentration in the tank and on the value of the target nitrogen loading rate. The pH is regulated by dosing the pH adjusting agent into the tank. The target nitrogen loading rate is determined in a laboratory apparatus according to the procedure described above, preferably its value is in the range of from 0.3 to 0.8 kg/(m³·d); the target operating pH value is then in the range of from 4.5 to 6.

In the most preferred embodiment, the target nitrogen loading rate is 0.5 kg/(m³ ·d) and the target operating pH value is 5.

For the first 10 days, the substrate (fugate) is dosed into the tank of the device with an intensity corresponding to 10 per cent of the target nitrogen loading rate value under conditions with pH regulated to the value of 7.

Subsequently, the system load will increase to 20 per cent of the target nitrogen loading rate value for 10 days and gradually to 40, 60 and 80 per cent of the target nitrogen loading rate value over the next ten-day periods. In this way, the nitrifying biomass cultivation device will reach its target performance after 50 days.

Simultaneously with the increase in the load of the device's tank, the operating pH value decreases. Once the target operating pH is reached, the effluent from the tank of the device can be used to inoculate a fully operating nitrification reactor according to the present invention to nitrify the fugate, which will be operated under analogous conditions.

The effluent from the nitrifying biomass cultivation device can be connected directly to the individual chambers S1, S2 and S3 of the nitrification reactor or it can be collected in suitable tanks (e.g. in IBC containers with a volume of 1 m$^3$). It is recommended to ensure aeration of IBC containers to achieve an oxygen concentration of at least 1 mg/L in said container.

Further object of the present invention is the use of said nitrification reactor to nitrify raw fugate from biogas plants.

Brief description of Drawings

Figure 1: Basic configuration of the nitrification reactor for fugate processing.

Figure 2: Division of the reactor for fugate nitrification into individual chambers S1, S2 and S3 having different volumes.

Figure 3: Division of the reactor for fugate nitrification with a total volume of 120 m$^3$ into three chambers of different volumes (Example 2)

Figure 4: Division of the reactor for fugate nitrification with a total volume of 400 m$^3$ into three chambers of different volumes (Example 4)

Figure 5: Scheme of nitrifying biomass cultivation device H

Examples

In the following text, we present two examples of the specific situation prevailing in the biogas plant (hereinafter referred to as BGP), in which a nitrification reactor for fugate processing is installed and incorporated.

*Method of determination of target nitrogen load and of target pH*

The target nitrogen loading rate and the target pH are determined by laboratory simulation of the nitrification process in a small laboratory nitrification reactor (volume of approx. 1 to 5 L). The nitrification process is initiated by placing an inoculum (e.g. activated sludge from a municipal wastewater treatment plant) in the laboratory nitrification reactor. The inoculum fills the entire reactor volume. Subsequently, the continuous dosing of the fugate into the nitrification reactor is started with a gradual increase of the fugate flow rate, resp. the reactor nitrogen loading rate. The nitrogen loading rate can be calculated as the product of the nitrogen concentration in the substrate (fugate) multiplied by the substrate (fugate) flow rate divided by the tank volume. The usual values of the target nitrogen loading rate for fugate are in the range of from 0.3 to 0.8 kg/(m$^3$ ·d), and the target operating pH value is in the range of from 4.5 to 6.

The pH value is continuously monitored in the reactor using a suitable measuring and control system. First, the fugate is dosed in an amount corresponding to the nitrogen loading rate of 0.05 kg/(m$^3$·d). In this phase of operation, with the use of the mentioned measuring and control system, there is a controlled decrease of the operating pH value, which is adjusted by dosing 10% NaOH solution. The dosing of this solution compensates for the decrease in pH caused by the release of H$^+$ ions during nitrification. Thus, within the controlled decrease of the operating pH value, the dosing intensity of the NaOH solution is gradually reduced. The purpose of this phase of reactor operation is to determine the target pH value. For the first 10 days, the operating pH is adjusted to 7. Subsequently, it is gradually reduced to 6.5 in subsequent ten-day periods; 6; 5.5; 5 and 4.5. The controlled pH reduction is terminated in the phase when the real pH value measured in the reactor is higher than the value set by the measuring and control system. This moment is also signaled by the fact that the dosing of the NaOH solution stops. The target pH identification phase ends at this point, the target pH value is considered to be the value applied in the period preceding the period when the pH value was applied at which the described situation was observed.

Subsequently, in the further operation of the laboratory reactor, the nitrogen loading rate is gradually increased to 0.1 in ten-day periods; 0.2; 0.3; 0.6; 0.8; 1.2; 1.6 and 2.0 kg/(m$^3$·d) to identify the target nitrogen loading rate (Bv). The pH is still continuously monitored and maintained at the target pH value by dosing 10% NaOH solution. The concentration of TAN in the reactor is monitored at regular 3-4 day intervals using standard analytical procedures. The increase of the reactor nitrogen loading rate is terminated when the reactor shows signs of overload (increase in pH above the target value and the associated absence of NaOH dosing, or increase in the concentration of TAN in the reactor above 200 mg/L). The target loading rate is then the value of the loading rate applied in the stage of its increase preceding the stage, in which the reactor was overloaded.

Example 1: *Construction of device H for nitrifying biomass cultivation*

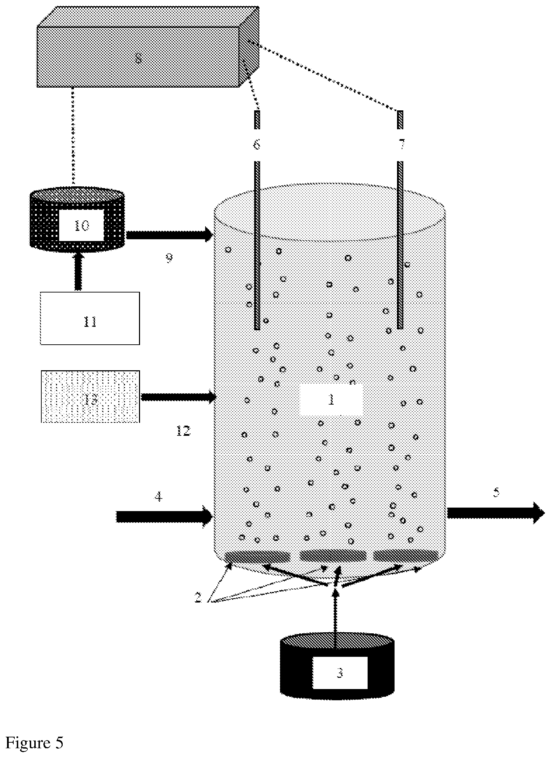

A device H for cultivation of nitrifying biomass was constructed, the scheme of which is shown in Fig. 5. The mobile unit of the nitrifying biomass cultivation device capable of processing for example fugate, can be operated, for example, as a system described below. Tank 1 of the device used in the mobile unit was made of plastic (polypropylene, PP) and has the shape of a cylinder with an inner diameter of 1000 mm. The outer diameter is 1012 mm; the total height of the cylinder is 1800 mm. The maximum working volume of the tank is 1 m$^3$. The pH value and the dissolved oxygen concentration can be measured and regulated using a measuring and control system consisting of a probe 6 for measuring pH, a probe 7 for measuring O$_2$ concentration, and

a measuring and control unit 8 (interface and the control unit itself (GRYF XBP; GRYF HB, spol. s r.o., Czech Republic)). According to the required pH value, the system can switch on the peristaltic pump 10 (PCD 21; Kouril dávkovací cerpadla, Czech Republic) with aqueous NaOH solution (40% by weight), which ensures that the required pH value in tank 1 is reached. The dosing of the substrate (fugate) into the tank 1 is provided by a peristaltic pump (PCD 1082; Kouril dávkovací cerpadla, Czech Republic). The dosing of fugate is continuous. The system works on the principle of a chemostat, i.e. without recirculation of nitrifying biomass. The air ensuring aerobic conditions and homogenization of the mixture in the reactor is continuously blown into the system by means of a source 3 of compressed air, in this case by means of a diaphragm blower capable of delivering 500 L of air per minute (Secoh JDK-S-500; Secoh, Japan). Even distribution of air bubbles in the entire volume of the reactor is ensured by three circular aeration elements 2 with a diameter of 23 cm with an air permeability of from 0 to 9.5 $m^3$/h (Edi FlexAir Disc 9" Micro; Environmental Dynamics Inc., USA).

The device H described above was used to inoculate the reactor for nitrification of all fugate production with a total volume of 120 $m^3$ in the building of an agricultural biogas plant with an installed electrical capacity of 0.5 MW and a daily fugate production of 20 $m^3$ at an average TAN concentration of 3.0 g/L. The target nitrogen loading rate of the nitrification reactor was set at 0.5 kg/($m^3$·d). Initially, fugate was dosed into tank 1 of the device, filled with activated sludge from the municipal wastewater treatment plant, with an intensity corresponding to 10% of the value of target nitrogen loading rate which means 0.05 kg/($m^3$ d) under conditions with pH adjusted to 7. This meant an initial flow rate of about 17 L per day. In this mode, the device operated for 10 days. Subsequently, the system loading rate increased to approx. 0.10 kg/($m^3$·d) over a period of 10 days and gradually to 0.20; 0.30; 0.40 and 0.50 kg/($m^3$ ·d) over additional ten-day periods. This meant a gradual increase in daily flow to 34, 68, 102, 136 and 170 L per day. Thus, after 50 days, the target reactor nitrogen loading rate of approx. 0.5 kg/($m^3$ ·d) was reached. Simultaneously with the increase in the loading rate of the tank 1 of the device, the operating pH value was gradually decreased. Ten days after the start of dosing the fugate into the device, the pH value decreased from 7 to 6.7, after another ten days to 6.5; after another ten days to 6 and finally after another 10 days to the pH value of 5. Once this was achieved, the effluent from tank 1 of the device could be used to inoculate the full scale nitrification reactor for fugate treatment. The outlet 5 from the tank 1 was collected in an IBC container and used in the following examples

Example 2: *Construction of the nitrification reactor I*

The aim was to realize the nitrification of all fugate production in the BGP with an installed electrical capacity of 0.5 MW and a daily fugate production of 20 $m^3$ at an average TAN concentration in the fugate of 3.0 g/L. Laboratory tests were performed to determine the target nitrogen load and the target pH. A laboratory simulation of the process was performed in a small reactor with a volume of 5 L, during which the conditions varied (fugate flowrate and thus nitrogen loading rate, pH, $O_2$ concentration) and the efficiency of the nitrification process was checked (the proportion of nitrified nitrogen in the effluent fugate was determined). The maximum nitrogen loading rate and minimum pH enabling to reach required processs efficiency were then used as the target nitrogen loading rate and operating pH value for the nitrification reactor (the method of their determination is described in the chapter "Method of determination of target nitrogen load and of target pH").

Laboratory tests showed that the optimal target nitrogen load of the nitrification reactor was 0.5 kg/($m^3$·d). A value of 4.5 was identified as the optimal operating pH. The incorporation of the operating device capable of processing all daily production of the fugate took place as follows:

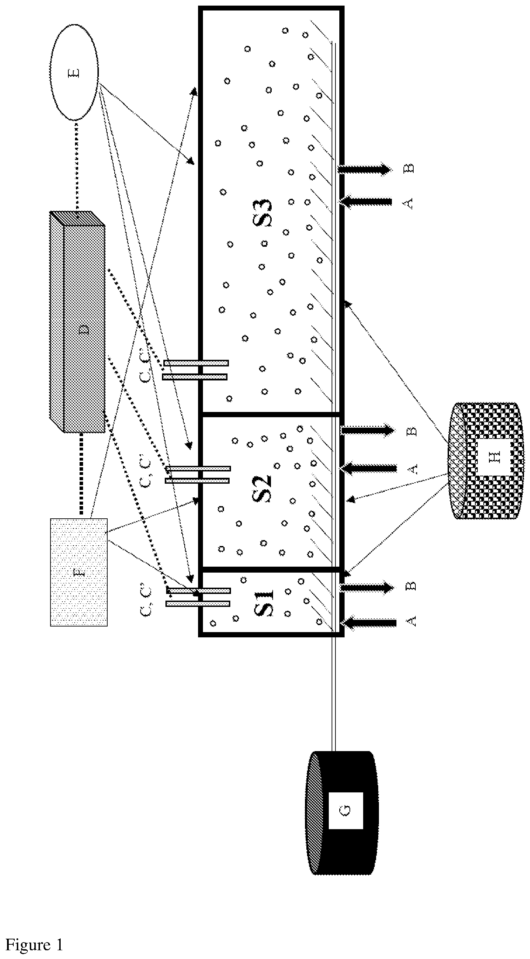

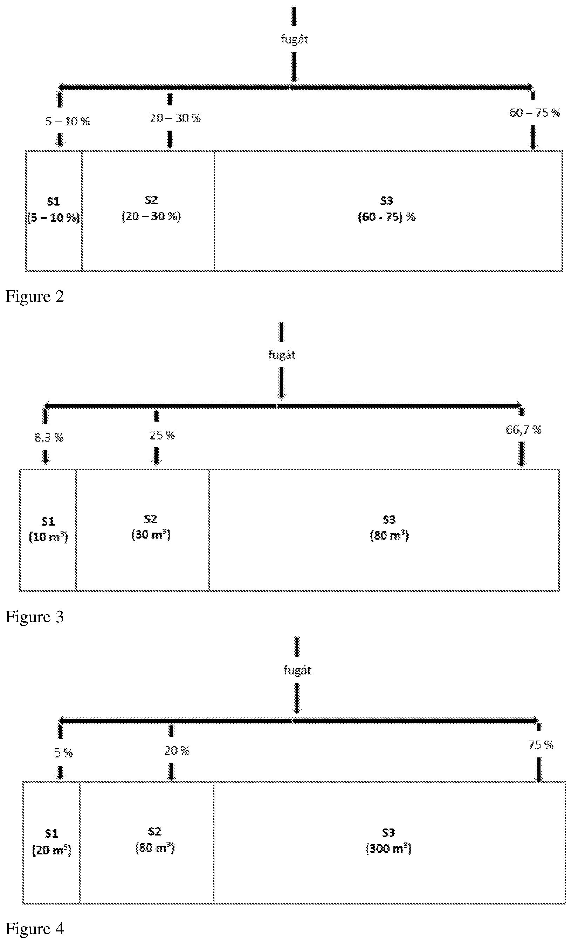

The concentration of TAN in the fugate was 3.0 g/L and the target nitrogen loading rate of the system is to reach 0.5 kg/($m^3$·d), so it will be necessary to achieve a hydraulic retention time in the reactor of 6 days. The reactor must therefore accommodate the six-day fugate production. The hydraulic retention time ($\Theta$) in days is calculated as the ratio of the TAN concentration and the target nitrogen loading rate (according to the following formula: $\Theta = c/Bv$). It therefore follows that the reactor must have a volume of 120 $m^3$. Thus, a nitrification reactor was built with tank 1 dimensions of 10 m x 3 m x 4 m (length x width x depth). The tank was made in the shape of a block, the material was concrete (the diagram of the reactor is in Figure 1). The reactor tank was divided into three chambers with a volume of 10 $m^3$ (S1), 30 $m^3$ (S2) and 80 $m^3$ (S3), see Figure 3.

An air distribution device G was connected to each of the tank chambers (S1, S2, S3), connected to a source of compressed air (blower) and used to aerate all the tank chambers. The blower was connected to the measuring and control unit D, located outside the tank.

Inlet A for the crude (raw) fugate was introduced into each of the tank chambers (S1, S2, S3), as was outlet B for the nitrified fugate.

Inside each chamber, a probe C for measuring pH, and a probe C' for measuring the $O_2$ concentration were placed, and both probes were connected to the measuring and control unit D. Then, an inlet for defoamer connected to a pump connected to the container F for defoamer was introduced into each of the chambers (S1, S2, S3).

A neutralization plant E comprising a pH adjusting agent tank (NaOH solution) and a pump was located outside the tank, and the neutralization plant E was connected to each chamber through a pH adjusting agent inlet.

The pump, neutralization station $\underline{E}$ and air distribution device $\underline{G}$ were connected to the measuring and control unit $\underline{D}$ by feedback.

Example 3: *Start-up of nitrification reactor I of Example 2*

After filling up the entire volume of the IBC container with the effluent from the mobile unit of the nitrifying biomass cultivation device $\underline{H}$ (see Example 1), the entire content of the IBC container, i.e. 1 m$^3$, was pumped to S1. At the same time, the output $\underline{5}$ from nitrifying biomass cultivation device $\underline{H}$ (Figure 5) was introduced into S1 (flow rate approx. 0.17 m$^3$/d), as well as the raw fugate feed through inlet $\underline{A}$, also in an amount corresponding to a flow rate of 0.17 m$^3$/d. Thus, at the start of the process in S1, a nitrogen loading rate analogous to that in the nitrifying biomass cultivation device $\underline{H}$ was achieved (and at the same time as the target loading rate of the full scale nitrification reactor, i.e. 0.5 kg/(m$^3\cdot$d), and with increasing volume of mixture in S1, the loading rate was gradually reduced, making it possible to gradually increase the flow rate of raw fugate. After five days, the volume of the mixture in S1 was about 2.7 m$^3$ and the flow rate of raw fugate could be increased to about 0.46 m$^3$/d. After another 5-day period, the daily flow of crude fugate could be increased to 0.80 m$^3$/d. At this stage, chamber S1 was completely filled up.

At the moment when this occurred, all the liquid contained in S 1 was pumped into S2 and the gradual filling of the chamber S2 with raw fugate started. In the following five-day periods, the daily flow of raw fugate could be gradually increased to 1.4 and 2.4 m$^3$, while at the end of the period with a daily flow of 2.4 m$^3$, S2 was completely filled up.

At the moment when this occurred, all the liquid contained in S2 was pumped into S3 and the gradual filling of the chamber S3 with raw fugate started. This was followed by two more five-day periods, during which the raw fugate flow gradually increased to 4.2 and 7.2 m$^3$ per day, while during the period with a daily flow of 7.2 m$^3$, S3 with a volume of 80 m$^3$ was completely filled up. In this phase, the total capacity of the operating reactor (120 m$^3$) was filled by two thirds.

At the time this occurred, part of the mixture from S3 was transferred to S1 and S2 so that the volume of liquid in all chambers represented 2/3 of their total capacity. The chamber S1 therefore contained about 6.7 m$^3$, section S2 20 m$^3$ and section S3 about 53.3 m$^3$ of processed fugate. From this moment on, the raw fugate was fed to the individual chambers via inlets $\underline{A}$ in an amount corresponding to their share in the total volume of the full scale reactor. Thus, about 8.3 per cent of the processed amount was fed to S1, about 25 per cent to S2 and about 66.7 per cent to S3 of the total amount of raw fugate fed to the system. At the same time, the flow rate of processed fugate continued to increase, and in the following five-day periods it gradually increased to 12.5 and 20.0 m$^3$ per day. Thus, after 45 days from the start of filling S1, the processing of all daily production of fugate was achieved. Once all chambers were filled, outlet $\underline{B}$ of the nitrified fugate from the reactor became the final outlet usable for application to soil. Throughout the process of start-up of the full scale reactor, outlet 5 from the nitrification biomass cultivation device $\underline{H}$ according to Example 1 was also added to the full scale reactor in order to enrich the system with nitrifying bacteria. This outlet $\underline{5}$, from the moment the chamber S3 was filled up and its volume divided into all three chambers, also opened into all three chambers and its dosed amount (volume) was distributed between the chambers in the same ratio as the volume ratio of these chambers. After all the chambers of the nitrification reactor tank had been filled up, this stream could already be stopped, because the amount of nitrifying organisms coming with it to the full scale reactor was already negligible in terms of the total volume of the full scale reactor.

Example 4: *Construction of the nitrification reactor II*

The aim was to realize the nitrification of all fugate production in the BGP with an installed electrical capacity of 1.5 MW and a daily fugate production of 60 m$^3$ at an average TAN concentration in the fugate of 5.0 g/L, with the need to nitrify 2/3 of the total daily production of fugate, i.e. 40 m$^3$ per day.

Laboratory tests showed that the optimal target nitrogen loading rate of the nitrification reactor was 0.5 kg/(m$^3$ d). A value of 5.5 was identified as the optimal operating pH value. The start-up of the full scale reactor capable of processing the required part of the daily production of the fugate took place as follows:

If the concentration of TAN in the fugate is 5.0 g/L and the target nitrogen loading rate of the system is to reach 0.5 kg/ (m$^3\cdot$d), it will be necessary to achieve a hydraulic retention time in the reactor of 10 days, i.e. the reactor must accommodate the ten-day fugate production.

The hydraulic retention time ($\Theta$) in days is calculated as the ratio of the TAN concentration and the target nitrogen loading rate ($\Theta$ = c/Bv).

It therefore follows that the reactor must have a volume of 400 m$^3$. A nitrification reactor of Figure 1 was built with tank $\underline{1}$ dimensions of 20 m x 5 m x 4 m (length x width x depth). The reactor was divided into three chambers with a volume of 20 m$^3$ (S1), 80 m$^3$ (S2) and 300 m$^3$ (S3), see Figure 1 and 4.

An air distribution device $\underline{G}$ was connected to each of the tank chambers, connected to a source of compressed air (blower) and used to aerate all the tank chambers. The blower was connected to the measuring and control unit $\underline{D}$ located outside the tank by feedback.

Inlet $\underline{A}$ for the raw fugate was introduced into each of the tank chambers ($\underline{S1}$, $\underline{S2}$, $\underline{S3}$), as was outlet $\underline{B}$ for the nitrified fugate.

Inside each chamber, a probe $\underline{C}$ for measuring pH, and a probe $\underline{C}'$ for measuring the $O_2$ concentration

were placed, and both probes were connected to the measuring and control unit D. Then, an inlet for defoamer connected to a pump connected to the container F for defoamer was introduced into each of the chambers (S1, S2, S3).

A neutralization plant E comprising a pH adjusting agent tank (Ca(OH)$_2$ solution) and a pump was located outside the tank, and the neutralization plant E was connected to each chamber (S1, S2, S3) through a pH adjusting agent inlet.

The pump, neutralization station E and air distribution device G were connected to the measuring and control unit D by feedback.

Example 5: *Start-up of nitrification reactor II of Example 4*

After filling up the entire volume of two IBC container with the effluent from the nitrifying biomass cultivation device H (see Example 1), the entire content of those IBC containers, i.e. 2 m$^3$, was pumped to S1.

At the same time, output 5 from device H of Example 1 (flow approx. 0.10 m$^3$/d) and raw fugate in an amount corresponding to a flow of 0.20 m$^3$/d were introduced into S1. Thus, at the start of the process in S1, a nitrogen loading rate analogous to that in device H according to Example 1 (and at the same time as the target loading rate of the operating reactor, i.e. 0.5 kg/(m$^3$·d)) was achieved; and with increasing volume of mixture in S1, the loading rate was gradually reduced, making it possible to gradually increase the flow rate of raw fugate. After five days, the volume of the mixture in S1 was about 3.5 m$^3$ and the flow rate of raw fugate could be increased to about 0.35 m$^3$/d. After further five-day periods, the daily flow rate of raw fugate could be gradually increased to 0.55; 0.90 and 1.40 m$^3$/d. At this stage, chamber S1 was filled up. At the moment when this occurred, all the liquid contained in S1 was pumped into S2 and the gradual filling of the chamber S2 started. In further five-day periods, the daily flow rate of raw fugate could be gradually increased to 2.1; 3.2; 4.9 and 7.4 m$^3$, and at the end of the period with a daily flow of 7.4 m$^3$, S2 was completely filled up. At the moment when this occurred, all the liquid contained in S2 was pumped into S3 and the gradual filling of the chamber S3 started. This was followed by two more five-day periods, during which the raw fugate flow gradually increased to 11.1; 16.7 and 25.1 m$^3$ per day, while during the period with a daily flow of 25.1 m$^3$, S3 with a volume of 300 m$^3$ was completely filled up. In this phase, the total capacity of the full scale reactor (120 m$^3$) was 75% full.

At the moment when this occurred, part of the mixture was transferred from S3 to S1 and S2 so that the volume of liquid in all chambers represented 75 per cent of their total capacity. Thus, there was about 15 m$^3$ of the processed fugate in chamber S1, 60 m$^3$ in chamber S2 and about 225 m$^3$ in chamber S3. From this moment on, the raw fugate was fed to the individual chambers (S1, S2, S3) in an amount corresponding to their share in the total volume of the operating reactor. Thus, about 5 per cent of the processed amount was fed to S1, about 20 per cent to S2 and about 75 per cent to S3 of the total amount of raw fugate fed to the system. At the same time, the flow rate of fugate to be processed continued to increase, gradually increasing to 37 and 40.0 m$^3$ per day in the following five-day periods. Thus, after 65 days from the start of filling S1, the processing of the required amount of fugate was achieved. Once all chambers (S1, S2, S3) were filled up, outlet B from the nitrification reactor became the final outlet usable for application to soil.

Throughout the entire phase of start-up of the nitrification reactor, outlet 5 from the nitrification biomass cultivation device H according to Example 1 was also added to the full scale reactor in order to enrich the system with nitrifying bacteria. This outlet 5, from the moment the chamber S3 was filled up and its volume divided into all three chambers, also opened into all three chambers (S1, S2, S3) and its dosed amount (volume) was distributed between the chambers in the same ratio as the volume ratio of these chambers. After the filling of the whole volume of full scale reactor is completed (or more precisely after the filling of the whole volume of all particular chambers of the reactor is completed), this stream could already be stopped, because the amount of nitrifying organisms coming with it to the full scale reactor was already negligible in terms of the total volume of the fully operating reactor.

*List of reference signs*

     A - inlet for raw fugate
     B - outlet for nitrified fugate
     C - probe for measuring pH
     C'- probe for measuring O$_2$ concentration
     D - measuring and control unit
     E - neutralization plant
     F - defoamer container
     G - air distribution device
     H - nitrifying biomass cultivation device
     1 - tank
     2 - aeration elements
     3 - source of compressed air
     4 - inlet for substrate
     5 - outlet for nitrifying biomass
     6 - probe for measuring pH
     7 - probe for measuring O$_2$ concentration
     8 - measuring and control unit
     9 - inlet for pH adjusting agent
     10 - pump
     11 - tank for pH adjusting agent
     12 -inlet for defoamer
     13 -container for defoamer

**Claims**

1. A multi-chamber nitrification reactor for fugate processing, **characterized in that** it comprises:

   - a tank which comprises three separate chambers (S1, S2, S3), wherein the chambers have mutually different volumes, wherein the volume of the first chamber (S1) is smaller than the volume of the second chamber (S2), which is smaller than the volume of the third chamber (S3), and wherein said tank further comprises an air distribution device (G) connected to a source of compressed air;
   - a measuring and control unit (D) located outside the tank;
   - an inlet (A) for raw fugate into each of the tank chambers (S1, S2, S3);
   - an outlet (B) for the nitrified fugate from each of the tank chambers (S1, S2, S3);
   - a probe (C) for measuring pH, located inside each chamber (S1, S2, S3) and connected to the measuring and control unit (D);
   - a probe (C') for measuring the $O_2$ concentration, located inside each chamber (S1, S2, S3) and connected to the measuring and control unit (D);
   - an inlet for a defoamer in each of the chambers (S1, S2, S3), connected to a pump connected to a container (F) for defoamer;
   - a neutralization station (E), located outside the tank, comprising a tank for the pH adjusting agent and a pump, said neutralization station (E) being connected to each chamber (S1, S2, S3) by an inlet for the pH adjusting agent; wherein the pump, and the air distribution device (G) are connected to the measuring and control unit (D) by feedback; and wherein the volume of the first chamber (S1) is in the range of from 5 to 10 % of the total reactor tank volume, the volume of the second chamber (S2) is in the range of from 20 to 30 % of the total reactor tank volume, and the volume of the third chamber (S3) is in the range of from 60 to 75 % of the total volume of the nitrification reactor tank; and wherein the device further comprises a pump configured to pump the liquid content from the first tank chamber (S1) to the second tank chamber (S2), and from the second tank chamber (S2) to the third tank chamber (S3).

2. The multi-chamber nitrification reactor according to claim 1, **characterized in that** the volume of the first chamber (S1) is 8.5 % of the total reactor tank volume, the volume of the second chamber (S2) is 25.5 % of the total reactor tank volume, and the volume of the third chamber (S3) is 66 % of the total volume of the nitrification reactor tank.

3. The multi-chamber nitrification reactor according to any one of the preceding claims, **characterized in that** the tank of the nitrification reactor is made of concrete or plastic and preferably has a volume in the range of from 30 to 500 $m^3$.

4. The multi-chamber nitrification reactor according to any one of the preceding claims, **characterized in that** each chamber (S1, S2, S3) further comprises an inlet for nitrifying biomass, adapted for connecting to an outlet (5) of a nitrifying biomass cultivation device (H), which comprises:

   - a tank (1), which comprises at least one aeration element (2), connected to a source (3) of compressed air;
   - measuring and control unit (8), located outside the tank (1);
   - an inlet (4) for substrate, located in the lower third of the height of the tank (1);
   - an outlet (5) for nitrifying biomass, preferably located in the upper third of the height of the tank (1);
   - a pH measuring probe (6), located inside the tank (1) and connected to the measuring and control unit (8);
   - an $O_2$ concentration measuring probe (7), located inside the tank (1) and connected to the measuring and control unit (8);
   - an inlet (9) for pH adjusting agent connected to a pump (10) connected to the pH adjusting agent tank (11);

   wherein the pump (10) and the aeration element (2) are connected to the measuring and control unit (8) by feedback.

5. A method of the start-up of the multi-chamber nitrification reactor according to any one of the preceding claims 1 to 4 for a given biogas plant, **characterized in that** it comprises the following steps:

   a) determination of the target nitrogen loading rate (Bv) and of the operating pH value of the multi-chamber nitrification reactor;
   b) calculating the reactor volume of the multi-chamber nitrification reactor according to equation (1):

   $$V = c \cdot Q/Bv \quad (1);$$

   wherein
   V is the volume of the multi-chamber nitrification reactor ($m^3$); Bv is the target nitrogen loading rate (kg/($m^3 \cdot$d)); c is the concentration of TAN in raw fugate (kg/m$^3$); Q is the target flow rate of fugate to be processed in the reactor (m$^3$/d);

c) constructing of the multi-chamber nitrification reactor according to any one of the preceding claims 1 to 5, the total tank volume of which was determined in step b);

d) a nitrifying culture of microorganisms capable of processing the fugate is placed in the first chamber (S 1) of the nitrification reactor tank, and the raw fugate produced by the given biogas plant is gradually fed into the first chamber (S 1) at a daily inflow rate of from 0.25 to 5 % of the total daily fugate production of the given biogas plant;

e) subsequently, the inflow rate of raw fugate dosed into the first chamber (S 1) is increased to the value of Q, determined by equation (2):

$$Q = Bv \cdot V/c \qquad (2),$$

wherein V is the current volume of the mixture in the given chamber (m$^3$); Bv is the target nitrogen loading rate (kg/(m$^3$·d)); c is the concentration of TAN in the fugate (kg/m$^3$) and Q is the inflow rate of fugate dosed into the chamber (m$^3$/d); wherein this inflow rate is maintained for five days;

f) repeating step e) in five-day cycles until the first chamber (S1) is completely filled;

g) all the mixture contained in the first chamber (S1) is pumped into the second chamber (S2), and the gradual filling of the second chamber (S2) begins with the inflow rate of raw fugate determined by the equation (2); wherein the inflow rate is increased in five-day cycles according to the equation (2) until the second chamber (S2) is completely filled;

h) all the mixture contained in the second chamber (S2) is pumped into the third chamber (S3), and the gradual filling of the third chamber (S3) begins with the inflow rate of raw fugate determined by the equation (2); wherein the inflow rate is increased in five-day cycles according to the equation (2) until the third chamber (S3) is completely filled;

i) a part of the mixture from the third chamber (S3) is transferred to the first chamber (S1) and the second chamber (S2), so that the percentage of volume of liquid in each of the chambers, related to the maximum volume of the particular chamber, is the same in all of the chambers (S1, S2, S3);

j) raw fugate is dosed simultaneously into all the chambers (S1, S2, S3) with the inflow dosing rates determined by the equation (2) for each of the chambers (S1, S2, S3); and wherein the inflow rate of raw fugate is increased in five-day cycles until the value corresponding to the target daily fugate production of the given biogas plant is achieved, and thus the nitrification reactor is put into full operation.

6. The method of putting the multi-chamber nitrification reactor into operation according to claim 5, **characterized in that** the nitrifying culture of microorganisms capable of processing the fugate in step d) is obtained from a nitrifying biomass cultivation device (H), defined in claim 4, by a method comprising the following steps:

- the tank (1) of the nitrifying biomass cultivation device (H) is filled with inoculum;
- the target nitrogen loading rate and the target operating pH value are determined;
- subsequently, during 10 days, the substrate suitable for cultivation of nitrification biomass is dosed into the tank (1) of the device (H) via the inlet (4) for substrate, preferably using the peristaltic pump, with an intensity corresponding to 10 % of the target nitrogen loading rate value, with pH adjusted to 7, and with aeration using the aeration element (2);
- subsequently, the system loading rate is increased to 20 % of the target nitrogen loading rate value for 10 days, with pH adjusted to 6.7, and with aeration using the aeration element (2);
- subsequently, the system loading rate is increased to 40 % of the target nitrogen loading rate value for 10 days, with pH adjusted to 6.5, and with aeration using the aeration element (2);
- subsequently, the system loading rate is increased to 60 % of the target nitrogen loading rate value for 10 days, with pH adjusted to 6, and with aeration using the aeration element (2);
- subsequently, the system loading rate is increased to 80 % of the target nitrogen loading rate value for 10 days, with pH adjusted to the target operating pH value, and with aeration using the aeration element (2);

wherein the substrate suitable for cultivation of nitrification biomass is selected from the group comprising fugate, reject water formed during the dewatering of anaerobically digested sludge, landfill leachate and industrial wastewater with the content of TAN of at least 500 mg/L; and wherein the inoculum is preferably activated sludge collected in the biological stage of a municipal wastewater treatment plant.

7. The method of the start-up of the multi-chamber nitrification reactor according to claims 5 or 6, **characterized in that** simultaneously with dosing raw fugate into individual chambers of the nitrification reactor in steps d) through j), nitrifying biomass, produced by the nitrifying biomass cultivation device (H) defined in claim 4, is dosed into said chambers; preferably with the dosing rate in the range of from 0.10 m$^3$/d to 0,20 m$^3$/d.

8. Use of the multi-chamber nitrification reactor according to any one of the preceding claims 1 to 4 for nitrification of raw fugate from biogas plants.

**Patentansprüche**

1. Mehrkammer-Nitrifikationsreaktor zur Fugatverarbeitung, **dadurch gekennzeichnet, dass** er umfasst:

   - einen Tank, der drei separate Kammern (S1, S2, S3) umfasst, wobei die Kammern voneinander unterschiedliche Volumina aufweisen, wobei das Volumen der ersten Kammer (S1) kleiner ist als das Volumen der zweiten Kammer (S2), das kleiner ist als das Volumen der dritten Kammer (S3), und wobei der Tank ferner eine Luftverteilungsvorrichtung (G) umfasst, die mit einer Druckluftquelle verbunden ist;
   - eine außerhalb des Tanks angeordnete Mess- und Steuereinheit (D);
   - einen Einlass (A) für Rohfugat in jede der Tankkammern (S1, S2, S3);
   - einen Auslass (B) für das nitrifizierte Fugat aus jeder der Tankkammern (S1, S2, S3);
   - eine Sonde (C) zur Messung des pH-Werts, die sich in jeder Kammer (S1, S2, S3) befindet und mit der Mess- und Steuereinheit (D) verbunden ist;
   - eine Sonde (C') zur Messung der O$_2$-Konzentration, die sich in jeder Kammer (S1, S2, S3) befindet und mit der Mess- und Steuereinheit (D) verbunden ist;
   - einen Einlass für einen Entschäumer in jeder der Kammern (S1, S2, S3), verbunden mit einer Pumpe, die mit einem Behälter (F) für Entschäumer verbunden ist;
   - eine Neutralisationsstation (E), die sich außerhalb des Tanks befindet und einen Tank für das pH-Einstellmittel und eine Pumpe umfasst, wobei die Neutralisationsstation (E) mit jeder Kammer (S1, S2, S3) über einen Einlass für das pH-Einstellmittel verbunden ist;
   wobei die Pumpe und die Luftverteilungsvorrichtung (G) durch Rückkopplung mit der Mess- und Steuereinheit (D) verbunden sind;
   und wobei das Volumen der ersten Kammer (S1) im Bereich von 5 bis 10 % des gesamten Reaktortankvolumens liegt, das Volumen der zweiten Kammer (S2) im Bereich von 20 bis 30 % des gesamten Reaktortankvolumens und das Volumen der dritten Kammer (S3) liegt im Bereich von 60 bis 75 % des gesamten Nitrifikationsreaktortankvolumens;
   und wobei die Vorrichtung weiterhin eine Pumpe umfasst, die dazu konfiguriert ist, den flüssigen Inhalt von der ersten Tankkammer (S1) in die zweite Tankkammer (S2) und von der zweiten Tankkammer (S2) in die dritte Tankkammer (S3) zu pumpen.

2. Mehrkammer-Nitrifikationsreaktor nach Anspruch 1, **dadurch gekennzeichnet, dass** das Volumen der ersten Kammer (S1) 8,5 % des gesamten Reaktortankvolumens beträgt, das Volumen der zweiten Kammer (S2) 25,5 % des gesamten Reaktortankvolumens beträgt, und das Volumen der dritten Kammer (S3) beträgt 66 % des gesamten Nitrifikationsreaktortankvolumens.

3. Mehrkammer-Nitrifikationsreaktor nach einem der vorhergehenden Ansprüche, **dadurch gekennzeichnet, dass** der Tank des Nitrifikationsreaktors aus Beton oder Kunststoff besteht und vorzugsweise ein Volumen im Bereich von 30 bis 500 m$^3$ aufweist.

4. Mehrkammer-Nitrifikationsreaktor nach einem der vorhergehenden Ansprüche, **dadurch gekennzeichnet, dass** jede Kammer (S1, S2, S3) ferner einen Einlass für nitrifizierende Biomasse aufweist, der zum Anschluss an einen Auslass (5) des Nitrifikationsbiomassekultivierungsgeräts (H) geeignet ist, das umfasst:

   - einen Tank (1), der mindestens ein Belüftungselement (2) umfasst, das an eine Druckluftquelle (3) angeschlossen ist;
   - außerhalb des Tanks (1) angeordnete Mess- und Steuereinheit (8);
   - einen Einlass (4) für Substrat, der sich im unteren Drittel der Höhe des Tanks (1) befindet;
   - einen Auslass (5) für nitrifizierende Biomasse, vorzugsweise im oberen Drittel der Höhe des Tanks (1) angeordnet;
   - eine pH-Messsonde (6), die sich im Tank (1) befindet und mit der Mess- und Steuereinheit (8) verbunden ist;
   - eine O$_2$-Konzentrationsmesssonde (7), die sich im Tank (1) befindet und mit der Mess- und Steuereinheit (8) verbunden ist;
   - einen Einlass (9) für pH-Einstellmittel, verbunden mit einer Pumpe (10), die mit dem pH-Einstellmitteltank (11) verbunden ist;

   wobei die Pumpe (10) und das Belüftungselement (2) durch Rückkopplung mit der Mess- und Steuereinheit (8) verbunden sind.

5. Verfahren zur Inbetriebnahme des Mehrkammer-Nitrifikationsreaktors nach einem der vorhergehenden Ansprüche 1 bis 4 für eine gegebene Biogasanlage, **dadurch gekennzeichnet, dass** es folgende Schritte umfasst:

a) Bestimmung der angestrebten Stickstoffbeladungsrate (Bv) und des Betriebs-pH-Wertes des Mehrkammer-Nitrifikationsreaktors;

b) Berechnen des Reaktorvolumens des Mehrkammer-Nitrifikationsreaktors gemäß Gleichung (1):

$$V = c \cdot Q/Bv \qquad (1);$$

worin

V ist das Volumen des Mehrkammer-Nitrifikationsreaktors ($m^3$); Bv ist die angestrebte Stickstoffbeladungsrate (kg/($m^3 \cdot$d)); c ist die TAN-Konzentration im Rohfugat (kg/$m^3$); Q ist die Zieldurchflussrate des im Reaktor zu verarbeitenden Fugats ($m^3$/d);

c) Aufbau des Mehrkammer-Nitrifikationsreaktors nach einem der vorhergehenden Ansprüche 1 bis 5, dessen Gesamttankvolumen in Schritt b) bestimmt wurde;

d) in der ersten Kammer (S1) des Nitrifikationsreaktortanks wird eine nitrifizierende Kultur von Mikroorganismen, die das Fugat verarbeiten können, platziert und das von der jeweiligen Biogasanlage produzierte Rohfugat wird nach und nach in die erste Kammer (S1) eingespeist mit täglicher Zuflussrate von 0,25 bis 5 % der gesamten täglichen Fugatproduktion der jeweiligen Biogasanlage;

e) anschließend wird die Zuflussrate des in die erste Kammer (S1) dosierten Rohfugats auf den durch Gleichung (2) bestimmten Wert Q erhöht:

$$Q = Bv \cdot V/c \qquad (2),$$

wobei V das aktuelle Volumen der Mischung in der gegebenen Kammer ($m^3$) ist; Bv ist die angestrebte Stickstoffbeladungsrate (kg/($m^3$·d)); c ist die TAN-Konzentration im Fugat (kg/$m^3$) und Q ist die Zuflussrate des in die Kammer dosierten Fugats ($m^3$/d); wobei diese Zuflussrate fünf Tage lang aufrechterhalten wird;

f) Wiederholen von Schritt e) in Fünf-Tages-Zyklen, bis die erste Kammer (S1) vollständig gefüllt ist;

g) das gesamte in der ersten Kammer (S1) enthaltene Gemisch wird in die zweite Kammer (S2) gepumpt und die allmähliche Befüllung der zweiten Kammer (S2) beginnt mit der durch die Gleichung (2) bestimmten Zuflussrate des Rohfugats; wobei die Zuflussrate in Fünf-Tages-Zyklen gemäß Gleichung (2) erhöht wird, bis die zweite Kammer (S2) vollständig gefüllt ist;

h) das gesamte in der zweiten Kammer (S2) enthaltene Gemisch wird in die dritte Kammer (S3) gepumpt und die allmähliche Befüllung der dritten Kammer (S3) beginnt mit der durch die Gleichung (2) bestimmten Zuflussrate des Rohfugats; wobei die Zuflussrate in Fünf-Tages-Zyklen gemäß Gleichung (2) erhöht wird, bis die dritte Kammer (S3) vollständig gefüllt ist;

i) ein Teil der Mischung aus der dritten Kammer (S3) wird in die erste Kammer (S 1) und die zweite Kammer (S2) überführt, so dass sich der prozentuale Volumenanteil der Flüssigkeit in jeder der Kammern, bezogen auf das Maximalvolumen der jeweiligen Kammer, ist in allen Kammern (S1, S2, S3) gleich;

j) Rohfugat wird gleichzeitig in alle Kammern (S1, S2, S3) dosiert, wobei die Zuflussdosierungsraten durch die Gleichung (2) für jede der Kammern (S1, S2, S3) bestimmt werden; und wobei die Zuflussrate des Rohfugats in fünftägigen Zyklen erhöht wird, bis der Wert erreicht ist, der der angestrebten täglichen Fugatproduktion der jeweiligen Biogasanlage entspricht, und somit der Nitrifikationsreaktor in den Vollbetrieb genommen wird.

6. Verfahren zur Inbetriebnahme des Mehrkammer-Nitrifikationsreaktors nach Anspruch 5, **dadurch gekennzeichnet, dass** die nitrifizierende Kultur von Mikroorganismen, die in der Lage sind, das Fugat in Schritt d) zu verarbeiten, aus einem Nitrifikationsbiomassekultivierungsgerät (H), definiert in Anspruch 4, gewonnen wird durch ein Verfahren, das die folgenden Schritte umfasst:

- der Tank (1) des Nitrifikationsbiomassekultivierungsgeräts (H) wird mit Inokulum gefüllt;
- die angestrebte Stickstoffbeladungsrate und der angestrebte Betriebs-pH-Wert werden bestimmt;
- dann wird, innerhalb von 10 Tagen, das für den Anbau von Nitrifikationsbiomasse geeignete Substrat in den Tank des Nitrifikationsbiomassekultivierungsgeräts (H) dosiert über den Substrateinlass (4), vorzugsweise mit der Schlauchpumpe, mit einer Intensität entsprechend 10% des Zielwerts der Stickstoffbeladungsrate, mit auf 7 eingestelltem pH-Wert und mit Belüftung mithilfe des Belüftungselements (2);
- dann wird die Systembeladungsrate für 10 Tage auf 20% des Zielwerts der Stickstoffbeladungsrate erhöht, wobei der pH-Wert auf 6,7 eingestellt wird und die Belüftung mit dem Belüftungselement (2) erfolgt.

- dann wird die Systembeladungsrate für 10 Tage auf 40% des Zielwertes der Stickstoffbeladungsrate erhöht, wobei der pH-Wert auf 6,5 eingestellt wird und die Belüftung mit dem Belüftungselement (2) erfolgt.

- dann wird die Systembeladungsrate für 10 Tage auf 60% des Zielwerts der Stickstoffbeladungsrate erhöht, wobei der pH-Wert auf 6 eingestellt wird und die Belüftung mit dem Belüftungselement (2) erfolgt.

- dann wird die Systembeladungsrate für 10 Tage auf 80% des Zielwertes der Stickstoffbeladungsrate erhöht, wobei der pH-Wert auf den Ziel-pH-Wert für den Betrieb eingestellt wird und die Belüftung mithilfe des Belüftungselements erfolgt (2);

wobei das für den Anbau von Nitrifikationsbiomasse geeignete Substrat aus der Gruppe ausgewählt ist, die Fugat, bei der Entwässerung von anaerob vergärtem Schlamm gebildetes Schmutzwasser, Deponiesickerwasser und Industrieabwasser mit einem TAN-Gehalt von mindestens 500 mg/L umfasst;

und wobei das Inokulum vorzugsweise Belebtschlamm ist, der in der biologischen Stufe einer kommunalen Abwasseraufbereitungsanlage gesammelt wird.

7. Verfahren zur Inbetriebnahme des Mehrkammer-Nitrifikationsreaktors nach den Ansprüchen 5 oder 6, **dadurch gekennzeichnet, dass** gleichzeitig mit der Dosierung von Rohfugat in einzelne Kammern des Nitrifikationsreaktors in den Schritten d) bis j), nitrifizierende Biomasse, erzeugt durch das in Anspruch 4 definiertes Nitrifikationsbiomassekultivierungsgerät (H), in die Kammern dosiert wird; vorzugsweise mit einer Dosierungsrate im Bereich von 0,10 $m^3$/Tag bis 0,20 $m^3$/Tag.

8. Verwendung des Mehrkammer-Nitrifikationsreaktors nach einem der vorhergehenden Ansprüche 1 bis 4 zur Nitrifikation von Rohfugat aus Biogasanlagen.

**Revendications**

1. Un réacteur de nitrification multi-chambres pour traitement des fugates, **caractérisé en ce qu'**il comprend :

- un réservoir qui comprend trois chambres séparées (S1, S2, S3), dans lequel les chambres ont des volumes mutuellement différents, dans lequel le volume de la première chambre (S1) est plus petit que le volume de la deuxième chambre (S2), qui est plus petit que le volume de la troisième chambre (S3), et dans lequel ledit réservoir comprend en outre un dispositif (G) de distribution d'air connecté à une source d'air comprimé ;

- une unité (D) de mesure et de contrôle située à l'extérieur du réservoir ;

- une entrée (A) pour le fugate brut dans chacune des chambres (S1, S2, S3) du réservoir ;

- une sortie (B) pour le fugate nitrifié de chacune des chambres (S1, S2, S3) du réservoir ;

- une sonde (C) de mesure du pH, située à l'intérieur de chaque chambre (S1, S2, S3) et reliée à l'unité (D) de mesure et de contrôle ;

- une sonde (C') de mesure de la concentration en $O_2$, située à l'intérieur de chaque chambre (S1, S2, S3) et reliée à l'unité (D) de mesure et de contrôle ;

- une entrée pour antimousse dans chacune des chambres (S1, S2, S3), reliée à une pompe reliée à un récipient (F) pour antimousse ;

- un poste (E) de neutralisation, situé à l'extérieur du réservoir, comprenant un réservoir pour l'agent d'ajustement du pH et une pompe, ledit poste (E) de neutralisation étant relié à chaque chambre (S1, S2, S3) par une entrée pour l'agent d'ajustement du pH;

dans lequel la pompe et le dispositif (G) de distribution d'air sont connectés à l'unité (D) de mesure et de commande par rétroaction ;

et dans lequel le volume de la première chambre (S1) est entre 5 et 10 % du volume total du réservoir du réacteur, le volume de la seconde chambre (S2) est entre 20 et 30 % du volume total du réservoir du réacteur, et le volume de la troisième chambre (S3) est entre 60 et 75 % du volume total du réservoir du réacteur de nitrification ;

et le dispositif comprenant en outre une pompe configurée pour pomper le contenu liquide de la première chambre (S1) de réservoir à la deuxième chambre (S2) de réservoir, et de la deuxième chambre (S2) de réservoir à la troisième chambre (S3) de réservoir.

2. Le réacteur de nitrification multi-chambres selon la revendication 1, **caractérisé en ce que** le volume de la première chambre (S1) est de 8,5 % du volume total du réservoir du réacteur, le volume de la deuxième chambre (S2) est de 25,5 % du volume total de la cuve du réacteur, et le volume de la troisième chambre (S3) est de 66 % du volume total du réservoir du réacteur de nitrification.

3. Le réacteur de nitrification multichambre selon l'une quelconque des revendications précédentes, **caractérisé en ce que** le réservoir du réacteur de nitrification est en béton ou en plastique et a de préférence son volume est entre 30 et 500 $m^3$.

**4.** Le réacteur de nitrification multi-chambres selon l'une quelconque des revendications précédentes, **caractérisé en ce que** chaque chambre (S1, S2, S3) comprend en outre une entrée pour la biomasse nitrifiante, adaptée pour se connecter à une sortie (5) d'un dispositif (H) de culture de biomasse nitrifiante, qui comprend :

- un réservoir (1), qui comprend au moins un élément d'aération (2), relié à une source (3) d'air comprimé ;
- une unité (8) de mesure et de contrôle, située à l'extérieur du réservoir (1) ;
- une entrée (4) pour substrat, située dans le tiers inférieur de la hauteur du réservoir (1) ;
- une sortie (5) de nitrification de la biomasse, située de préférence dans le tiers supérieur de la hauteur du réservoir (1) ;
- une sonde (6) de mesure du pH, située à l'intérieur du réservoir (1) et reliée à l'unité (8) de mesure et de contrôle ;
- une sonde (7) de mesure de concentration en $O_2$, située à l'intérieur du réservoir (1) et reliée à l'unité (8) de mesure et de contrôle;
- une entrée (9) pour agent d'ajustement du pH reliée à une pompe (10) reliée au réservoir (11) d'agent d'ajustement du pH;

dans lequel la pompe (10) et l'élément (2) d'aération sont connectés à l'unité (8) de mesure et de commande par rétroaction.

**5.** Un procédé de démarrage du réacteur de nitrification multi-chambre selon l'une quelconque des revendications 1 à 4 précédentes pour une usine de biogaz, **caractérisé en ce qu'**il comprend les étapes suivantes :

a) détermination du taux de charge d'azote cible (Bv) et de la valeur du pH de fonctionnement du réacteur de nitrification multi-chambres;
b) calcul du volume du réacteur de nitrification multi-chambre selon l'équation (1) :

$$V = c \cdot Q/Bv \qquad (1) ;$$

dans laquelle
V est le volume du réacteur de nitrification multi-chambre ($m^3$) ; Bv est le taux cible de chargement en azote (kg/($m^3$.d)) ; c est la concentration de TAN dans le fugat brut (kg/$m^3$) ; Q est le débit cible du fugat à traiter dans le réacteur ($m^3$/d) ;
c) construction du réacteur de nitrification multi-chambre selon l'une quelconque des revendications 1 à 5 précédentes, dont le volume total du réservoir a été déterminé à l'étape b) ;

d) une culture nitrifiante de micro-organismes capables de traiter le fugat est placée dans la première chambre (S1) du réservoir du réacteur de nitrification, et le fugat brut produit par l'usine de biogaz est progressivement introduit dans la première chambre (S1) à un taux d'entrée quotidien compris entre 0,25 et 5 % de la production quotidienne totale de fugat de l'usine de biogaz ;
e) par la suite, le débit d'entrée du fugat brut dosé dans la première chambre (S 1) est augmenté jusqu'à la valeur Q, déterminée par l'équation (2) :

$$Q = Bv \cdot V/c \qquad (2),$$

où V est le volume actuel du mélange dans la chambre donnée ($m^3$) ; Bv est le taux cible de chargement en azote (kg/($m^3$.d)) ; c est la concentration de TAN dans le fugat (kg/$m^3$) et Q est le débit d'entrée du fugat dosé dans la chambre ($m^3$/d) ;
dans lequel ce débit d'entrée est maintenu pendant cinq jours ;

f) répéter l'étape e) par cycles de cinq jours jusqu'à ce que la première chambre (S1) soit complètement remplie ;
g) tout le mélange contenu dans la première chambre (S1) est pompé dans la deuxième chambre (S2), et le remplissage progressif de la deuxième chambre (S2) commence avec le débit d'entrée du fugat brut déterminé par l'équation (2) ; le débit d'entrée est augmenté par cycles de cinq jours conformément à l'équation (2) jusqu'à ce que la deuxième chambre (S2) soit complètement remplie;
h) tout le mélange contenu dans la deuxième chambre (S2) est pompé dans la troisième chambre (S3), et le remplissage progressif de la troisième chambre (S3) commence avec le débit d'entrée du fugat brut déterminé par l'équation (2) ; le débit d'entrée est augmenté par cycles de cinq jours selon l'équation (2) jusqu'à ce que la troisième chambre (S3) soit complètement remplie ;
i) une partie du mélange de la troisième chambre (S3) est transférée dans la première chambre (S 1) et la deuxième chambre (S2), de sorte que le pourcentage du volume de liquide dans chacune des chambres, par rapport au volume maximal de la chambre en question, soit le même dans toutes les chambres (S1, S2, S3) ;
j) le fugate brut est dosé simultanément dans toutes les chambres (S1, S2, S3) avec les taux de dosage d'entrée déterminés par l'équation (2) pour chacune des chambres (S1, S2, S3) ;
et dans lequel le taux d'entrée de fugate brut est augmenté par cycles de cinq jours jusqu'à ce que la valeur correspondant à la production quotidienne cible de fugate de l'usine de biogaz donnée soit atteinte, et ainsi le réacteur de nitrification est mis en marche.

6. Le procédé de démarrage du réacteur de nitrification multi-chambre selon la revendication 5, **caractérisé en ce que** la culture nitrifiante de micro-organismes capables de traiter le fugate à l'étape d) est obtenue à partir du dispositif (H) de culture de biomasse nitrifiante, défini en revendication 4, par un procédé comprenant les étapes suivantes :

- le réservoir (1) du dispositif (H) de culture de biomasse nitrifiante est remplie d'inoculum ;
- le taux de charge en azote cible et la valeur de pH de fonctionnement cible sont déterminés ;
- ensuite, pendant 10 jours, le substrat adapté à la culture de biomasse nitrifiée est dosé dans le réservoir (1) du dispositif (H) via l'entrée (4) pour substrat, de préférence à l'aide de la pompe péristaltique, avec une intensité correspondant à 10 % de la valeur cible du taux de charge en azote, avec un pH ajusté à 7, et avec une aération à l'aide de l'élément (2) d'aération;
- ensuite, le taux de charge du système est augmenté à 20 % de la valeur cible du taux de charge en azote pendant 10 jours, avec un pH ajusté à 6,7, et avec une aération à l'aide de l'élément (2) d'aération;
- ensuite, le taux de charge du système est augmenté jusqu'à 40 % de la valeur cible du taux de charge en azote pendant 10 jours, avec un pH ajusté à 6,5, et avec une aération à l'aide de l'élément (2) d'aération;
- ensuite, le taux de charge du système est augmenté à 60 % de la valeur cible du taux de charge en azote pendant 10 jours, avec un pH ajusté à 6, et avec une aération à l'aide de l'élément (2) d'aération;
- ensuite, le taux de charge du système est augmenté jusqu'à 80 % de la valeur cible du taux de charge en azote pendant 10 jours, avec un pH ajusté à la valeur de pH de fonctionnement cible, et avec une aération à l'aide de l'élément (2) d'aération;
le substrat approprié pour la culture de biomasse nitrifiée étant choisi dans le groupe comprenant le fugate, les eaux rejetées formées lors de la déshydratation de boues digérées de manière anaérobie, les lixiviats de décharge et les eaux usées industrielles ayant une teneur en TAN d'au moins 500 mg/L ;
et dans lequel l'inoculum est de préférence une boue activée collectée au stade biologique d'une usine de traitement des eaux usées municipales.

7. Le procédé de démarrage du réacteur de nitrification multi-chambres selon les revendications 5 ou 6, **caractérisé en ce que** simultanément au dosage du fugate brut dans les chambres individuelles du réacteur de nitrification aux étapes d) à j), la biomasse nitrifiante, produite par le dispositif (H) de culture de biomasse nitrifiante défini dans la revendication 4, est dosé dans lesdites chambres ; de préférence avec un débit de dosage compris entre 0,10 $m^3$/d et 0,20 $m^3$/d.

8. Utilisation du réacteur de nitrification multi-chambres selon l'une quelconque des revendications 1 à 4 précédentes pour la nitrification du fugate brut provenant d'usines de biogaz.

Figure 1

fugát

5 – 10 %   20 – 30 %   60 – 75 %

| S1<br>(5 – 10 %) | S2<br>(20 – 30 %) | S3<br>(60 - 75) % |

Figure 2

fugát

8,3 %   25 %   66,7 %

| S1<br>(10 m³) | S2<br>(30 m³) | S3<br>(80 m³) |

Figure 3

fugát

5 %   20 %   75 %

| S1<br>(20 m³) | S2<br>(80 m³) | S3<br>(300 m³) |

Figure 4

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 106520610 A

**Non-patent literature cited in the description**

- **SVEHLA et al.** *Journal of Environmental Management,* 2020, vol. 276, 111250

- **SVEHLA et al.** *Waste Management.,* 2017, vol. 64, 96-106